# EUROPÄISCHE PATENTSCHRIFT

(11) **EP 2 032 553 B1**
(45) Veröffentlichungstag und Bekanntmachung des Hinweises auf die Patenterteilung: **16.12.2009**
(21) Anmeldenummer: 07730041.6
(22) Anmeldetag: 11.06.2007
(51) Int. Cl.: C07D 323/06

(54) **INTEGRIERTES VERFAHREN ZUR HERSTELLUNG VON TRIOXAN AUS FORMALDEHYD**
INTEGRATED METHOD FOR THE PREPARATION OF TRIOXANE FROM FORMALDEHYDE
PROCÉDÉ INTÉGRÉ DE PRÉPARATION DE TRIOXANNE À PARTIR DE FORMALDÉHYDE

(30) Priorität: 12.06.2006 EP 06115287
(43) Veröffentlichungstag der Anmeldung: 11.03.2009
(73) Patentinhaber: BASF SE, 67056 Ludwigshafen (DE)
(72) Erfinder: SIEGERT, Markus, 69126 Heidelberg (DE); LANG, Neven, 68163 Mannheim (DE); STRÖFER, Eckhard, 68163 Mannheim (DE); SIGWART, Christoph, 69469 Weinheim (DE)
(86) Internationale Anmeldenummer: PCT/EP2007/055694
(87) Internationale Veröffentlichungsnummer: WO 2007/144320

(56) Entgegenhaltungen:
- WO-A-2005/063353
- WO-A-2005/063733
- WO-A-2007/017410
- WO-A-2007/017479

## Beschreibung

Die Erfindung betrifft ein integriertes Verfahren zur Herstellung von Trioxan aus Formaldehyd.

Trioxan wird in der Regel durch Reaktivdestillation von wässriger Formaldehydlösung in Gegenwart saurer Katalysatoren hergestellt. Dabei fällt ein Trioxan, Formaldehyd und Wasser enthaltendes Gemisch als Destillat an. Aus diesem Gemisch wird anschließend das Trioxan durch Extraktion mit halogenierten Kohlenwasserstoffen, wie Methylenchlorid oder 1,2-Dichlorethan, oder anderen, mit Wasser nicht mischbaren Lösungsmitteln abgetrennt.

W02005063733 beschreibt ein Verfahren zur Abtrennung von Trioxan aus einem Einsatzstrom (I) aus Formaldehyd, Trioxan und Wasser, bei dem a) ein Einsatzstrom (I), der als Hauptkomponente Formaldehyd und als Nebenkomponenten Trioxan und Wasser enthält, bereitgestellt wird, b) der Einsatzstrom (I) mit einem Rückführstrom (VII), der als Hauptkomponente Trioxan und als Nebenkomponenten Formaldehyd und Wasser enthält, gemischt wird, wobei ein Einsatzstrom (la), der als Hauptkomponente Formaldehyd und als Nebenkomponenten Trioxan und Wasser enthält, erhalten wird, c) der Einsatzstrom (la) in einer ersten Destillationsstufe bei einem Druck von 0,1 bis 2,5 bar destilliert wird, wobei ein Strom (II), der als Hauptkomponente Formaldehyd und als Nebenkomponente Wasser enthält, und ein Strom (III), der als Hauptkomponente Trioxan und als Nebenkomponenten Wasser und Formaldehyd enthält, erhalten werden, d) der Strom (III), gegebenenfalls nach Abtrennung von Leichtsiedern aus dem Strom (III) in einer Leichtsieder-Abtrennstufe, in einer zweiten Destillationsstufe bei einem Druck von 0,2 bis 17,5 bar destilliert wird, wobei der Druck in der zweiten Destillationsstufe um 0,1 bis 15 bar höher als der Druck in der ersten Destillationsstufe ist, wobei ein Strom (IV), der im wesentlichen aus Trioxan besteht, und ein Strom (V), der als Hauptkomponente Trioxan und als Nebenkomponenten Wasser und Formaldehyd enthält, erhalten wird, e) gegebenenfalls der Strom (V) mit einem Strom (IX), der als Hauptkomponente Wasser enthält, gemischt wird, wobei ein Strom Va mit höherem Wassergehalt als Strom (V), wobei der Strom (Va) als Hauptkomponenten Trioxan und als Nebenkomponenten Wasser und Formaldehyd enthält, erhalten wird, f) der Strom (V) bzw. (Va) in einer dritten Destillationsstufe bei einem Druck von 1 bis 10 bar destilliert wird, wobei ein Strom (VI), der im wesentlichen aus Wasser besteht, und der Rückführstrom (VII), der als Hauptkomponente Trioxan und als Nebenkomponenten Wasser und Formaldehyd enthält, erhalten werden.

DE-A 1 668 867 beschreibt ein Verfahren zur Abtrennung von Trioxan aus Wasser, Formaldehyd und Trioxan enthaltenden Gemischen durch Extraktion mit einem organischen Lösungsmittel. Dabei wird eine aus zwei Teilstrecken bestehende Extraktionsstrecke an einem Ende mit einem organischen, mit Wasser praktisch nicht mischbaren Extraktionsmittel für Trioxan beschickt, am anderen Ende mit Wasser. Zwischen den beiden Teilstrecken wird das zu trennende Destillat aus der Trioxan-Synthese zugeführt. Auf der Seite der Lösungsmittelzuführung wird dabei eine wässrige Formaldehydlösung und auf der Seite der Wasserzuführung eine praktisch formaldehydfreie Lösung von Trioxan in dem organischen Lösungsmittel erhalten.

Nachteilig an dieser Verfahrensweise ist der Anfall von Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrenstoffverordnung), deren Handhabung besondere Vorsichtsmaßnahmen erfordert.

DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in reines Trioxan einerseits und ein azeotropes Gemisch aus Trioxan, Wasser und Formaldehyd andererseits auftrennt. In einem Beispiel wird ein wässriges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trio-xan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt.

Diese Verfahrensweise ist sehr aufwändig. Insbesondere die Pervaporationseinheit erfordert hohe Investitionen.

Aufgabe der Erfindung ist es, ein alternatives Verfahren zur Herstellung von Trioxan aus wässriger Formaldehydlösung unter Gewinnung von reinem Trioxan bereitzustellen. Aufgabe ist es insbesondere, ein Verfahren bereitzustellen, welches die Durchführung von Extraktionsschritten oder Pervaporationsschritten zur Gewinnung von reinem Trioxan vermeidet.

Gelöst wird die Aufgabe durch ein integriertes Verfahren zur Herstellung von Trioxan aus Formaldehyd mit den Schritten:
a) ein Formaldehyd und Wasser enthaltender Einspeisungsstrom A1 und ein überwiegend Wasser und daneben Formaldehyd und Trioxan enthaltender Rückführstrom B3 werden einer Formaldehyd-Aufkonzentrierungseinheit zugeführt und in einen Formaldehyd-reichen Strom A2 und einen im Wesentlichen aus Wasser bestehenden Strom A3 aufgetrennt;
b) ein Trioxan und Wasser enthaltender Produktstrom C1, ein Trioxan und Wasser enthaltender Rückführstrom E1 und gegebenenfalls der Formaldehyd-reiche Strom A2 werden einer ersten Niederdruck-Destillationskolonne zugeführt und bei einem Druck von 0,1 bis 1,5 bar destilliert, wobei ein an Trioxan angereicherter, überwiegend Trioxan und daneben Wasser und Formaldehyd enthaltender Strom B1, ein im Wesentlichen aus Formaldehyd und Wasser bestehender Sumpfabzugsstrom B2 und der überwiegend Wasser und daneben Formaldehyd und Trioxan enthaltende Rückführstrom B3 als Seitenabzugsstrom entnommen werden;
c) der Sumpfabzugsstrom B2 und gegebenenfalls der Strom A2 werden einem Trioxan-Synthesereaktor zugeführt und reagieren gelassen, wobei der Trioxan, Wasser und Formaldehyd enthaltende Strom C1 erhalten wird;
d) der Strom B1 wird einer Mitteldruck-Destillationskolonne zugeführt und bei einem Druck von 1,0 bis 4,0 bar destilliert, wobei ein Leichtsiederstrom D1 enthaltend Methanol, Methylal und Methylformiat und ein überwiegend Trioxan und daneben Formaldehyd und Wasser enthaltender Strom D2 erhalten werden;
e) der Strom D2 wird einer Hochdruck-Destillationskolonne zugeführt und bei einem Druck von 2,0 bis 10,0 bar destilliert, wobei der Trioxan und Wasser enthaltende Rückführstrom E1 und ein im Wesentlichen aus Trioxan bestehender Produktstrom E2 erhalten werden;
wobei der Strom A2 entweder der Niederdruck-Destillationskolonne oder dem Trioxan-Synthesereaktor oder beiden zugeführt werden kann.

"Im Wesentlichen bestehend aus" soll vorstehend und nachfolgend bedeuten, dass der betreffende Strom zu mindestens 80 Gew.-%, vorzugsweise zu mindestens 90 Gew.-% aus den genannten Komponenten besteht. "Überwiegend enthaltend" soll bedeuten, dass die genannte Komponente in dem Strom die Hauptkomponente darstellt, bevorzugt in diesem zu mindestens 50 Gew.-% enthalten ist.

Es ist bekannt, dass Trioxan, Formaldehyd und Wasser ein ternäres Azeotrop bilden, welches bei einem Druck von 1 bar aus 69 Gew.-% Trioxan, 5 Gew.-% Formaldehyd und 26 Gew.-% Wasser besteht.

Erfindungsgemäß wird das ternäre Azeotrop durch eine Druckwechseldestillation getrennt, indem eine erste und eine zweite Destillationsstufe bei verschiedenen Drücken durchgeführt werden. In einer Niederdruck-Destillationsstufe wird aus einem an Trioxan und Formaldehyd reichen, wässrigen Gemisch ein trioxanreiches Trioxan/Wasser/Formaldehyd-Gemisch, welches nur noch wenig Formaldehyd enthält, abgetrennt. Das trioxanreiche Trioxan/Wasser/Formaldehyd-Gemisch wird in einer nachgeschalteten Hochdruck-Destillationsstufe in ein trioxanreiches Trioxan/Wasser/Formaldehyd-Gemisch einerseits und praktisch reines Trioxan andererseits aufgetrennt. Zwischen der Niederdruck- und der Hochdruck-Destillationsstufe ist zur Abtrennung von Leichtsiedern noch eine Mitteldruck-Destillationsstufe vorgesehen. Erfindungsgemäß wird weiterhin der Niederdruck-Destillationsstufe ein Formaldehyd/Wasser-Gemisch mit hohem Wasseranteil als Seitenabzug entnommen, welcher zur Wasser-Abtrennung gemeinsam mit dem wässrigen Formaldehyd-Einsatzstrom einer Formaldehyd-Aufkonzentrierungsstufe zugeführt wird.

Als Hochdruck-, Mitteldruck- beziehungsweise Niederdruck-Destillationskolonnen kommen beliebige Destillationskolonnen, wie Packungs- oder Bodenkolonnen, in Frage. Die Destillationskolonnen können beliebige Einbauten, Packungen oder Füllkörperschüttungen enthalten. Im Folgenden beziehen sich alle Druckangaben auf den Druck am Kopf der betreffenden Kolonne.

In einem ersten Verfahrensschritt a) werden ein Formaldehyd und Wasser enthaltender Einspeisungsstrom A1 und ein überwiegend Wasser und daneben Formaldehyd und Trioxan enthaltender Rückführstrom B3 einer Formaldehyd-Aufkonzentrierungseinheit zugeführt und in einen Formaldehyd-haltigen Strom A2 und einen im Wesentlichen aus Wasser bestehenden Strom A3 aufgetrennt.

Im Allgemeinen enthält der Einspeisungsstrom A1 20 bis 65 Gew.-% Formaldehyd und 35 bis 80 Gew.-% Wasser. Der Rückführstrom B3 enthält im Allgemeinen 15 bis 70 Gew.-% Wasser, 10 bis 50 Gew.-% Formaldehyd und 1 bis 30 Gew.-% Trioxan. Der Formaldehyd-reiche Strom A2 enthält im Allgemeinen 45 bis 75 Gew.-% Formaldehyd, 20 bis 55 Gew.-% Wasser und 0,1 bis 15 Gew.-% Trioxan. Der im Wesentlichen aus Wasser bestehende Strom A3 enthält im Allgemeinen 90 bis 100 Gew.-% Wasser und daneben noch in geringen Mengen Formaldehyd und Ameisensäure sowie andere üblicherweise bei der Trioxan-Synthese entstehenden Komponenten, wie z. B. Dimethoxydimethylether und Trimethoxydimethylether, beispielsweise in Mengen von insgesamt bis zu 10,0 Gew.-%.

Die Aufkonzentrierung a) des Formaldehyd/Wasser-Gemischs erfolgt bei einem Druck von im Allgemeinen 0,1 bis 10,0 bar, bevorzugt in einer Druckdestillationskolonne bei einem Druck von im Allgemeinen 1,0 bis 10,0 bar, wobei am Kolonnensumpf ein wässriger Strom, der im Wesentlichen aus Wasser besteht, abgezogen wird. Eine solche Kolonne kann beispielsweise bei einem Druck von 5,5 bar, einer Kopftemperatur von 147 °C und einer Sumpftemperatur von 156 °C betrieben werden.

In einem Verfahrenschritt b) werden ein Trioxan, Wasser und Formaldehyd enthaltender Produktstrom C1, der in einem Trioxan-Synthesereaktor erzeugt wird, ein Trioxan und Wasser enthaltender Rückführstrom E1 und gegebenenfalls der Formaldehyd-reiche Strom A2 aus der Aufkonzentrierungs-Einheit einer ersten Niederdruck-Destillationskolonne zugeführt. Der Strom A2 kann auch ganz oder teilweise direkt dem Trioxan-Synthesereaktor zugeführt werden.

Vorzugsweise wird der Niederdruck-Destillationskolonne der Strom E1 als Seitenzulauf im oberen Drittel der Kolonne, beispielsweise 1 bis 20 theoretische Böden unterhalb des Kolonnenkopfes, und der Strom C1 als Seitenzulauf im unteren Drittel der Kolonne, vorzugsweise 1 bis 20 theoretische Böden oberhalb des Kolonnensumpfes, zugeführt. Wird der Strom A2 ganz oder teilweise der Niederdruck-Kolonne zugeführt, so erfolgt dies im Allgemeinen im unteren Drittel der Kolonne, vorzugsweise 1 bis 15 theoretische Böden oberhalb des Sumpfes.

Die Niederdruckkolonne wird im Allgemeinen bei einem Druck von 0,1 bis 1,5 bar, vorzugsweise im Vakuum bei einem Druck von 0,5 bis 0,9 bar betrieben. Es fallen ein an Trioxan angereicherter, überwiegend Trioxan und daneben Wasser und Formaldehyd enthaltender Strom B1, vorzugsweise als Kopfabzugsstrom, ein im Wesentlichen aus Formaldehyd und Wasser bestehender Sumpfabzugsstrom B2 und der überwiegend Wasser und daneben Formaldehyd und Trioxan enthaltende Rückführstrom B3 als Seitenabzugsstrom an. Letzterer wird im Allgemeinen in der oberen Hälfte, vorzugsweise im oberen Drittel der Kolonne, entnommen. Optional kann der Niederdruck-Destillationskolonne vorzugsweise im unteren Drittel ein Seitenabzugsstrom B4, der Hochsieder enthält, entnommen werden, um Hochsieder aus dem Verfahren auszuschleusen.

Die Niederdruck-Destillationskolonne weist im Allgemeinen 2 bis 50, bevorzugt 4 bis 40 theoretische Stufen auf.

Der an Trioxan angereicherte Strom B1 enthält im Allgemeinen 50 bis 75 Gew.-% Trioxan, 1 bis 25 Gew.-% Formaldehyd und 10 bis 40 Gew.-% Wasser. Der Sumpfabzugsstrom B2 enthält im Allgemeinen 50 bis 95 Gew.-% Formaldehyd und 5 bis 40 Gew.-% Wasser. Daneben kann er Trioxan enthalten, im Allgemeinen in Mengen von 0 bis 10 Gew.-%, sowie gegebenenfalls geringe Mengen Ameisensäure.

In einem Verfahrenschritt c) werden der Sumpfabzugsstrom B2 und gegebenenfalls der Strom A2 einem Trioxan-Synthesereaktor zugeführt und reagieren gelassen, wobei der Trioxan und Wasser enthaltende Strom C1 erhalten wird. Der Strom A2 aus der Formaldehyd-Aufkonzentrierung kann ganz oder teilweise dem Trioxan-Synthesereaktor zugeführt werden.

In einer Ausführungsform des erfindungsgemäßen Verfahrens wird das Wasser/Formaldehyd-Gemisch in der Trioxan-Synthesestufe c) in Gegenwart von sauren Homogen- oder Heterogen-Katalysatoren, wie Ionenaustauscherharzen, Zeolithen, Schwefelsäure oder p-Toluolsulfonsäure, bei einer Temperatur von im Allgemeinen 70 bis 130 °C umgesetzt. Die Trioxan-Synthese kann in einem Festbett- oder Fließbettreaktor an einem heterogenen Katalysator, z. B. einem Ionenaustauscherharz oder Zeolith, durchgeführt werden.

Der Produktstrom C1 enthält im Allgemeinen Trioxan, Formaldehyd und Wasser als Hauptkomponenten und daneben die üblicherweise bei der Trioxan-Synthese gebildeten Leichtsieder und Hochsieder. Im Allgemeinen enthält er 0,1 bis 35 Gew.-% Trioxan.-Leichtsieder und Schwersieder können beispielsweise in Mengen von 0,1 bis 10,0 Gew.-% enthalten sein.

Vorzugsweise handelt es sich bei dem Produktstrom C1 um einen Dampfstrom, welcher der Niederdruck-Destillationskolonne in Sumpfnähe zugeführt wird. Zur Ausschleusung von Hochsiedern kann ein Teil des in dem Trioxan-Synthesereaktor enthaltenen Reaktionsgemischs von Zeit zu Zeit entnommen werden.

In einer Ausführungsform des erfindungsgemäßen Verfahrens werden die Trioxan-Synthesestufe c) und die Niederdruck-Destillationsstufe b) gemeinsam als Reaktivdestillation in einer Reaktionskolonne durchgeführt. Diese kann im Abtriebsteil ein KatalysatorFestbett aus einem heterogenen Katalysator enthalten. Alternativ kann die Reaktivdestillation auch in Gegenwart eines homogenen Katalysators durchgeführt werden, wobei ein saurer Katalysator zusammen mit dem Wasser/Formaldehyd-Gemisch im Kolonnensumpf vorliegt. Der Seitenabzugsstrom B3 wird dann der Reaktivdestillationskolonne an geeigneter Stelle im Verstärkungsteil entnommen.

In einem Verfahrenschritt d) wird der Strom B1 einer Mitteldruck-Destillationskolonne zugeführt und bei einem Druck von 1,0 bis 3,0 bar, bevorzugt 1,5 bis 2,5 bar destilliert, wobei ein Leichtsiederstrom D1, der im Allgemeinen Methanol, Methylal und Methylformiat enthält, und ein überwiegend Trioxan und daneben Formaldehyd und Wasser enthaltender Strom D2 erhalten werden. Der Strom D2 ist im Wesentlichen wie der Strom B1 zusammengesetzt, enthält jedoch im Wesentlichen keine Leichtsieder mehr.

Im Allgemeinen besteht der Leichtsiederstrom zu mindestens 25 Gew.-% aus Leichtsiedern, im Allgemeinen aus Methanol, Methylal und Methylformiat und gegebenenfalls weiteren Leichtsiedern. Der Leichtsiederstrom D1 wird im Allgemeinen als Kopfabzugsstrom gewonnen, der Strom D2 am Kolonnensumpf entnommen.

Die Mitteldruck-Destillationskolonne weist im Allgemeinen 2 bis 50, bevorzugt 4 bis 40 theoretische Stufen auf.

In einem weiteren Verfahrensschritt e) wird der Strom D2 einer Hochdruck-Destillationskolonne zugeführt und bei einem Druck von 2,0 bis 10,0 bar, bevorzugt 3,0 bis 6,0 bar destilliert, wobei der Trioxan und Wasser enthaltende Rückführstrom E1 und ein im Wesentlichen aus Trioxan bestehender Produktstrom E2 erhalten werden,

Der Rückführstrom E1 enthält im Allgemeinen 25 bis 75 Gew.-% Trioxan, 10 bis 50 Gew.-% Wasser und 1 bis 25 Gew.-% Formaldehyd. Der Trioxan-Strom E2 besteht im Allgemeinen zu mindestens 99,0 Gew.-%, bevorzugt zu mindestens 99,99 Gew.-% aus Trioxan. Daneben kann er Wasser und Ameisensäure, beispielsweise in Mengen > 0,001 Gew.-%, und Hochsieder enthalten.

Die Hochdruck-Destillationskolonne weist im Allgemeinen 2 bis 50, bevorzugt 4 bis 40 theoretische Stufen auf.

Vorzugsweise wird der Hochdruck-Destillationskolonne der Strom D2 als Seitenzulauf zugeführt, der Strom E1 als Kopfabzugsstrom entnommen und der Strom E2 als Sumpfabzugsstrom entnommen. Der Strom E2 kann auch als gasförmiger Seitenabzug zwischen Zulauf und Kolonnensumpf entnommen werden.

In einer besonders bevorzugten Ausführungsform des erfindungsgemäßen Verfahrens wird die Niederdruck-Destillation b) bei einem Druck von 0,5 bis 0,9 bar, die Mitteldruck-Destillation c) bei einem Druck von 1,5 bis 2,5 bar und die Hochdruck-Destillation d) bei einem Druck von 4,0 bis 6,0 bar durchgeführt.

In einer Ausführungsform des erfindungsgemäßen Verfahrens wird der Produktstrom E2 noch weiter aufgereinigt. Hierzu kann er einer Reindestillationskolonne zugeführt und destilliert werden, wobei ein Rein-Trioxan-Strom F1 und ein Trioxan und Hochsieder enthaltender Strom F2 erhalten werden. Der Strom F1 wird im Allgemeinen am Kolonnenkopf entnommen und kann aus reinem Trioxan mit einer Reinheit von > 99,9 Gew.-% bestehen. Der Strom F2 wird im Allgemeinen am Kolonnensumpf entnommen und enthält Hochsieder. Daneben enthält er Trioxan, im Allgemeinen mindestens 10, bevorzugt mindestens 50 Gew.-%. Vorzugsweise wird der Strom F2 in den Trioxan-Synthesereaktor zurückgeführt. Die Trioxan-Reindestillationskolonne weist im Allgemeinen 1 bis 40, bevorzugt 2 bis 20 theoretische Stufen auf und wird im Allgemeinen bei einem Druck von 0,1 bis 2,5 bar, bevorzugt 0,75 bis 1,5 bar betrieben.

Vor Einspeisung in die Reindestillationskolonne kann der Strom E2 über ein Adsorptionsbett geleitet werden, um Spuren von Wasser und Ameisensäure zu entfernen. Geeignete Adsorbentien sind beispielsweise Molsiebe, Ionentauscher, Zeolithe, Mordenithe und Silicagel sowie weitere dem Fachmann bekannte Adsorbentien.

Das erhaltene Rein-Trioxan, dessen Reinheit im Allgemeinen > 99,9 Gew.-%, bevorzugt > 99,99 Gew.-% beträgt, wird vorzugsweise zur Herstellung von Polyoxymethylen (POM) oder Polyoxymethylenderivaten wie Polyoxymethylendimethylether (POMDME) und Diaminodiphenylmethan (MDA) verwendet.

Die Erfindung wird durch das nachfolgende Beispiel näher erläutert.

### Beispiel

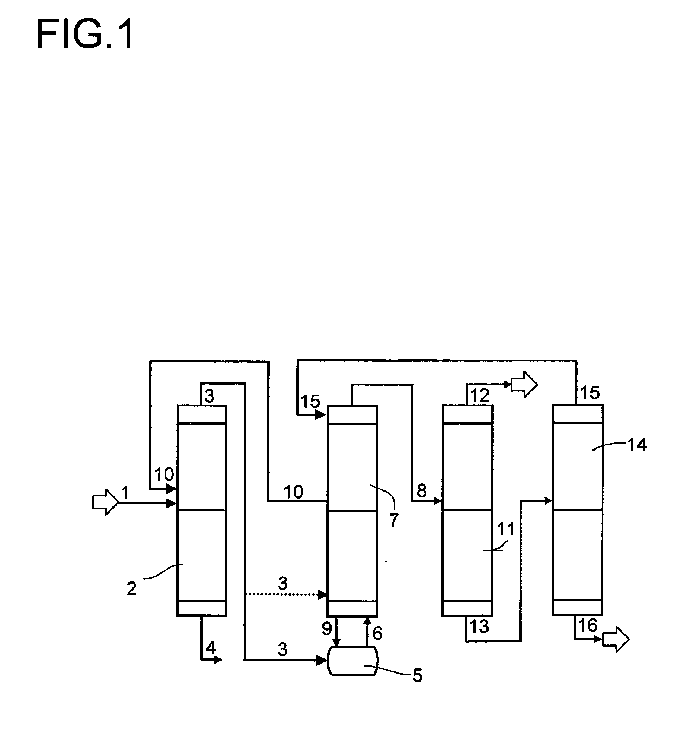

Die Figur zeigt eine Ausführungsform des erfindungsgemäßen Verfahrens. Es werden lediglich die Hauptkomponenten Formaldehyd, Wasser und Trioxan aufgezeigt. Ströme, deren Gehalt an diesen Hauptkomponenten in Summe kleiner 100 % ist, enthalten die üblichen bei der Trioxan-Synthese gebildeten Nebenkomponenten.

Der Druckdestillationskolonne 2 werden der Einspeisungsstrom 1 (= A1) aus 49 Gew.-% Formaldehyd und 51 Gew.-% Wasser und der Rückführstrom 10 (= B3) aus 22 Gew.-% Formaldehyd, 70 Gew.-% Wasser und 8 Gew.-% Trioxan zugeführt. Die Druckdestillationskolonne 2 wird bei einem Druck von 6 bar, einer Sumpftemperatur von 160 °C und einer Kopftemperatur von 148 °C betrieben. Als Kopfabzugsstrom 3 (= A2) wird ein Gemisch aus 54 Gew.-% Formaldehyd, 41 Gew.-% Wasser und 5 Gew.-% Trioxan erhalten. Als Sumpfabzugsstrom 4 (= A3) wird ein Abwasserstrom aus 98 Gew.-% Wasser, welcher noch geringe Mengen Ameisensäure (1,4 Gew.-%) und Formaldehyd (0,6 Gew.-%) enthält, erhalten. Der Kopfabzugsstrom 3 wird dem Trioxan-Synthesereaktor 5, welcher als Rührbehälter ausgebildet ist, zugeführt. Der Produktstrom 6 (= C1) enthält 25 Gew.-% Trioxan, 22 Gew.-% Wasser, 45 Gew.-% Formaldehyd und 5 Gew.-% Ameisensäure sowie geringe Mengen bei der Synthese gebildete Nebenkomponenten. Dieser Produktstrom 6 wird dampfförmig der Niederdruck-Destillationskolonne 7 mit 20 theoretischen Böden auf der Höhe des ersten theoretischen Bodens zugeführt. Weiterhin wird der Destillationskolonne 7 ein Rückführstrom 15 (= E1) aus 65 Gew.-% Trioxan, 31 Gew.-% Wasser und 4 Gew.-% Formaldehyd auf der Höhe des 19. theoretischen Bodens als Seitenzulauf zugeführt. Die Kolonne 7 wird bei einem Druck von 0,6 bar betrieben, die Sumpftemperatur beträgt ca. 93 °C, die Kopftemperatur ca. 62 °C. Es wird ein Kopfabzugsstrom 8 (= B1) aus 3 Gew.-% Formaldehyd, 26 Gew.-% Wasser und 70 Gew.-% Trioxan, und ein Sumpfabzugsstrom 9 (= B2) aus 65 Gew.-% Formaldehyd, 22 Gew.-% Wasser und 7 Gew.-% Trioxan erhalten. Letzterer wird in den Trioxan-Synthesereaktor 5 zurückgeführt. Ferner wird auf der Höhe des 14. theoretischen Bodens der Rückführstrom 10 (= B3) als Seitenabzugsstrom entnommen. Der Kopfabzugsstrom 8 wird der Mitteldruck-Destillationskolonne 11 mit 23 theoretischen Böden auf der Höhe des 5. theoretischen Bodens zugeführt. Die Kolonne 11 wird bei einem Druck von 1,6 bar betrieben, die Sumpftemperatur beträgt ca. 105 °C, die Kopftemperatur ca. 62 °C. Als Kopfabzugsstrom 12 (= D1) wird ein Gemisch aus 19 Gew.-% Methanol, 54 Gew.-% Methylal und 25 Gew.-% Methylformiat erhalten. Als Sumpfabzugsstrom 13 (= D2) wird ein Gemisch aus 3 Gew.-% Formaldehyd, 26 Gew.-% Wasser und 71 Gew.-% Trioxan erhalten. Der Sumpfabzugsstrom 13 wird der Hochdruck-Destillationskolonne 14 mit 24 theoretischen Böden auf der Höhe des 22. theoretischen Bodens zugeführt. Diese Kolonne wird bei 5 bar betrieben, die Sumpftemperatur beträgt ca. 176 °C, die Kopftemperatur ca. 143 °C. Es wird der Rückführstrom 15 (= E1) als Kopfabzugsstrom und ein Reinproduktstrom 16 (= E2) aus > 99,9 Gew.-% Trioxan als Sumpfabzugsstrom erhalten.

## Patentansprüche

1. Integriertes Verfahren zur Herstellung von Trioxan aus Formaldehyd mit den Schritten:
a) ein Formaldehyd und Wasser enthaltender Einspeisungsstrom A1 und ein Wasser als Hauptkomponente und daneben Formaldehyd und Trioxan enthaltender Rückführstrom B3 werden einer Formaldehyd-Aufkonzentrierungseinheit zugeführt und in einen Formaldehyd-reichen Strom A2 und einen mindestens 80 Gew.-% Wasser enthaltenden Strom A3 aufgetrennt;
b) ein Trioxan, Wasser und Formaldehyd enthaltender Produktstrom C1, ein Trioxan, Wasser und Formaldehyd enthaltender Rückführstrom E1 und gegebenenfalls der Strom A2 werden einer ersten Niederdruck-Destillationskolonne zugeführt und bei einem Druck von 0,1 bis 1,5 bar destilliert, wobei ein an Trioxan angereicherter, Trioxan als Hauptkomponente und daneben Wasser und Formaldehyd enthaltender Strom B1, ein mindestens 80 Gew.-% Formaldehyd und Wasser enthaltender Sumpfabzugsstrom B2 und der Wasser als Hauptkomponente und daneben Formaldehyd und Trioxan enthaltende Rückführstrom B3 als Seitenabzugsstrom entnommen werden;
c) der Sumpfabzugsstrom B2 und gegebenenfalls der Strom A2 werden einem Trioxan-Synthesereaktor zugeführt und reagieren gelassen, wobei der Trioxan, Wasser und Formaldehyd enthaltende Strom C1 erhalten wird;
d) der Strom B1 wird einer Mitteldruck-Destillationskolonne zugeführt und bei einem Druck von 1,0 bis 3,0 bar destilliert, wobei ein Leichtsiederstrom D1 enthaltend Methanol, Methylal und Methylformiat und ein überwiegend Trioxan und daneben Formaldehyd und Wasser enthaltender Strom D2 erhalten werden;
e) der Strom D2 wird einer Hochdruck-Destillationskolonne zugeführt und bei einem Druck von 2,5 bis 10,0 bar destilliert, wobei der Trioxan, Wasser und Formaldehyd enthaltende Rückführstrom E1 und ein mindestens 80 Gew.-% Trioxan enthaltender Produktstrom E2 erhalten werden;
wobei der Strom A2 entweder der Niederdruck-Destillationskolonne oder dem Trioxan-Synthesereaktor oder beiden zugeführt werden kann.

2. Verfahren nach Anspruch 1, **dadurch gekennzeichnet, dass** die Niederdruck-Destillation b) bei einem Druck von 0,1 bis 1,5 bar, die Mitteldruck-Destillation c) bei einem Druck von 1,0 bis 4,0 bar und die Hochdruck-Destillation d) bei einem Druck von 2,0 bis 10,0 bar durchgeführt werden.

3. Verfahren nach Anspruch 1 oder 2, **dadurch gekennzeichnet, dass** der Niederdruck-Destillationskolonne der Strom B1 als Kopfabzugsstrom entnommen wird.

4. Verfahren nach einem der Ansprüche 1 bis 3, **dadurch gekennzeichnet, dass** der Niederdruck-Destillationskolonne der Strom E1 als Seitenzulauf im oberen Drittel der Kolonne, 1 bis 20 theoretische Böden unterhalb des Kopfes, und der Strom C1 als Seitenzulauf im unteren Drittel der Kolonne, 1 bis 20 theoretische Böden oberhalb des Sumpfes, zugeführt werden.

5. Verfahren nach einem der Ansprüche 1 bis 4, **dadurch gekennzeichnet, dass** der Mitteldruck-Destillationskolonne der Strom D1 als Kopfabzugsstrom entnommen und der Strom D2 als Sumpfabzugsstrom entnommen werden.

6. Verfahren nach einem der Ansprüche 1 bis 5, **dadurch gekennzeichnet, dass** der Hochdruck-Destillationskolonne der Strom E1 als Kopfabzugsstrom entnommen und der Strom E2 als Sumpfabzugsstrom entnommen werden.

7. Verfahren nach einem der Ansprüche 1 bis 6, **dadurch gekennzeichnet, dass** in Schritt a) die Formaldehyd-Aufkonzentrierungseinheit eine Druckdestillationskolonne ist und der Strom A3 als wässriger Sumpfabzugsstrom entnommen wird.

8. Verfahren nach einem der Ansprüche 1 bis 6, **dadurch gekennzeichnet, dass** die Formaldehyd-Aufkonzentrierungseinheit ein Fallfilmverdampfer ist.

9. Verfahren nach einem der Ansprüche 1 bis 8, **dadurch gekennzeichnet, dass** der Niederdruck-Destillationskolonne im unteren Drittel, 1 bis 5 theoretische Böden oberhalb des Sumpfes, ein Seitenabzugsstrom B4, der Hochsieder enthält, entnommen wird.

10. Verfahren nach einem der Ansprüche 1 bis 8, **dadurch gekennzeichnet, dass** dem Trioxan-Synthesereaktor ein Strom B4, der Hochsieder enthält, entnommen wird.

11. Verfahren nach einem der Ansprüche 1 bis 10, **dadurch gekennzeichnet, dass** der Produktstrom E2 einer Reindestillationskolonne zugeführt und destilliert wird, wobei ein Rein-Trioxan-Strom F1 und ein Trioxan und Hochsieder enthaltender Strom F2 erhalten werden.

12. Verfahren nach Anspruch 11, **dadurch gekennzeichnet, dass** der Strom F2 in den Trioxan-Synthesereaktor zurückgeführt wird.

13. Verfahren nach Anspruch 11 oder 12, **dadurch gekennzeichnet, dass** der Strom E2 vor Einspeisung in die Reindestillationskolonne über ein Adsorptionsbett geleitet wird, wobei Spuren von Wasser und Ameisensäure aus dem Strom E2 entfernt werden.

## Claims

1. An integrated process for preparing trioxane from formaldehyde, comprising the steps of:
a) feeding a feed stream A1 comprising formaldehyde and water and a recycle stream B3 comprising water as the main component and additionally formaldehyde and trioxane to a formaldehyde concentration unit and separating it into a formaldehyde-rich stream A2 and a stream A3 comprising at least 80% by weight of water;
b) feeding a product stream C1 comprising trioxane, water and formaldehyde, a recycle stream E1 comprising trioxane, water and formaldehyde, and, if appropriate, the stream A2 to a first low-pressure distillation column and distilling at a pressure of from 0.1 to 1.5 bar, and withdrawing a trioxane-enriched stream B1 comprising trioxane as the main component comprising at least 80% by weight and additionally water and formaldehyde, a bottom draw stream B2 of formaldehyde and water, and the recycle stream B3 comprising water as the main component and additionally formaldehyde and trioxane as a side draw stream;
c) feeding the bottom draw stream B2 and, if appropriate, the stream A2 to a trioxane synthesis reactor and allowing them to react to obtain the stream C1 comprising trioxane, water and formaldehyde;
d) feeding the stream B1 to a medium-pressure distillation column and distilling at a pressure of from 1.0 to 3.0 bar to obtain a low boiler stream D1 comprising methanol, methylal and methyl formate, and a stream D2 comprising predominantly trioxane and additionally formaldehyde and water;
e) feeding the stream D2 to a high-pressure distillation column and distilling at a pressure of from 2.5 to 10.0 bar to obtain the recycle stream E1 comprising trioxane, water and formaldehyde, and a product stream E2 comprising at least 80% by weight of trioxane;
the stream A2 being fed either to the low-pressure distillation column or to the trioxane synthesis reactor or to both.

2. The process according to claim 1, wherein the low-pressure distillation b) is carried out at a pressure of from 0.1 to 1.5 bar, the medium-pressure distillation c) at a pressure of from 1.0 to 4.0 bar, and the high-pressure distillation d) at a pressure of from 2.0 to 10.0 bar.

3. The process according to claim 1 or 2, wherein stream B1 is withdrawn from the low-pressure distillation column as a top draw stream.

4. The process according to any of claims 1 to 3, wherein stream E1 is fed to the low-pressure distillation column as a side feed in the upper third of the column, from 1 to 20 theoretical plates below the top, and stream C1 as a side feed in the lower third of the column, from 1 to 20 theoretical plates above the bottom.

5. The process according to any of claims 1 to 4, wherein stream D1 is withdrawn from the medium-pressure distillation column as a top draw stream and stream D2 is withdrawn as a bottom draw stream.

6. The process according to any of claims 1 to 5, wherein stream E1 is withdrawn from the high-pressure distillation column as a top draw stream and stream E2 is withdrawn as a bottom draw stream.

7. The process according to any of claims 1 to 6, wherein, in step a), the formaldehyde concentration unit is a pressure distillation column and stream A3 is withdrawn as an aqueous bottom draw stream.

8. The process according to any of claims 1 to 6, wherein the formaldehyde concentration unit is a falling-film evaporator.

9. The process according to any of claims 1 to 8, wherein a side draw stream B4 which comprises high boilers is withdrawn from the low-pressure distillation column in the lower third, from 1 to 5 theoretical plates above the bottom.

10. The process according to any of claims 1 to 8, wherein a stream B4 which comprises high boilers is withdrawn from the trioxane synthesis reactor.

11. The process according to any of claims 1 to 10, wherein product stream E2 is fed to a purifying distillation column and distilled to obtain a pure trioxane stream F1 and a stream F2 comprising trioxane and high boilers.

12. The process according to claim 11, wherein stream F2 is recycled into the trioxane synthesis reactor.

13. The process according to claim 11 or 22, wherein stream E2, before it is fed into the purifying distillation column, is passed through an adsorption bed, which removes traces of water and formic acid from stream E2.

## Revendications

1. Procédé intégré de fabrication de trioxane à partir de formaldéhyde, comprenant les étapes suivantes :
a) un courant d'alimentation A1 contenant du formaldéhyde et de l'eau et un courant de recyclage B3 contenant de l'eau en tant que composant principal et également du formaldéhyde et du trioxane sont introduits dans une unité de concentration du formaldéhyde et séparés en un courant riche en formaldéhyde A2 et un courant A3 contenant au moins 80 % d'eau ;
b) un courant de produits C1 contenant du trioxane, de l'eau et du formaldéhyde, un courant de recyclage E1 contenant du trioxane, de l'eau et du formaldéhyde et éventuellement le courant A2 sont introduits dans une première colonne de distillation à pression basse et distillés à une pression de 0,1 à 1,5 bars, un courant B1 enrichi en trioxane, contenant du trioxane en tant que composant principal et également de l'eau et du formaldéhyde, un courant de sortie de fond B2 contenant au moins 80 % de formaldéhyde et de l'eau et le courant de recyclage B3 contenant de l'eau en tant que composant principal et également du formaldéhyde et du trioxane en tant que courant de sortie latérale étant soutirés ;
c) le courant de sortie de fond B2 et éventuellement le courant A2 sont introduits dans un réacteur de synthèse de trioxane et laissés réagir, le courant C1 contenant du trioxane, de l'eau et du formaldéhyde étant obtenu ;
d) le courant B1 est introduit dans une colonne de distillation à pression moyenne et distillé à une pression de 1,0 à 3,0 bars, un courant de composés de point d'ébullition bas D1 contenant du méthanol, du méthylal et du formiate de méthyle et un courant D2 contenant en majorité du trioxane et également du formaldéhyde et de l'eau étant obtenus ;
e) le courant D2 est introduit dans une colonne de distillation à pression élevée et distillé à une pression de 2,5 à 10,0 bars, le courant de recyclage E1 contenant du trioxane, de l'eau et du formaldéhyde et un courant de produits E2 contenant au moins 80 % en poids de trioxane étant obtenus ;
le courant A2 pouvant être introduit soit dans la colonne de distillation à pression basse, soit dans le réacteur de synthèse de trioxane, soit dans les deux.

2. Procédé selon la revendication 1, **caractérisé en ce que** la distillation à pression basse b) est réalisée à une pression de 0,1 à 1,5 bars, la distillation à pression moyenne c) à une pression de 1,0 à 4,0 bars et la distillation à pression élevée d) à une pression de 2,0 à 10,0 bars.

3. Procédé selon la revendication 1 ou 2, **caractérisé en ce que** le courant B1 est soutiré de la colonne de distillation à pression basse en tant que courant de sortie de tête.

4. Procédé selon l'une quelconque des revendications 1 à 3, **caractérisé en ce que** le courant E1 est introduit dans la colonne de distillation à pression basse en tant qu'alimentation latérale dans le tiers supérieur de la colonne, 1 à 20 plateaux théoriques en dessous de la tête, et le courant C1 en tant qu'alimentation latérale dans le tiers inférieur de la colonne, 1 à 20 plateaux théoriques au-dessus du fond.

5. Procédé selon l'une quelconque des revendications 1 à 4, **caractérisé en ce que** le courant D1 est soutiré de la colonne de distillation à pression moyenne en tant que courant de sortie de tête et le courant D2 en tant que courant de sortie de fond.

6. Procédé selon l'une quelconque des revendications 1 à 5, **caractérisé en ce que** le courant E1 est soutiré de la colonne de distillation à pression élevée en tant que courant de sortie de tête et le courant E2 en tant que courant de sortie de fond.

7. Procédé selon l'une quelconque des revendications 1 à 6, **caractérisé en ce qu'**à l'étape a), l'unité de concentration du formaldéhyde est une colonne de distillation sous pression et le courant A3 est soutiré en tant que courant de sortie de fond aqueux.

8. Procédé selon l'une quelconque des revendications 1 à 6, **caractérisé en ce que** l'unité de concentration du formaldéhyde est un évaporateur à film tombant.

9. Procédé selon l'une quelconque des revendications 1 à 8, **caractérisé en ce qu'**un courant de sortie latérale B4, qui contient des composés à point d'ébullition élevé, est soutiré de la colonne de distillation à pression basse dans le tiers inférieur, 1 à 5 plateaux théoriques au-dessus du fond.

10. Procédé selon l'une quelconque des revendications 1 à 8, **caractérisé en ce qu'**un courant B4, qui contient des composés à point d'ébullition élevé, est soutiré du réacteur de synthèse de trioxane.

11. Procédé selon l'une quelconque des revendications 1 à 10, **caractérisé en ce que** le courant de produits E2 est introduit dans une colonne de distillation pure et distillé, un courant F1 de trioxane pur et un courant F2 contenant du trioxane et des composés à point d'ébullition élevé étant obtenus.

12. Procédé selon la revendication 11, **caractérisé en ce que** le courant F2 est recyclé dans le réacteur de synthèse de trioxane.

13. Procédé selon la revendication 11 ou 12, **caractérisé en ce que** le courant E2 est passé sur un lit d'adsorption avant l'introduction dans la colonne de distillation pure, les traces d'eau et d'acide formique étant éliminées du courant E2.
